# EUROPEAN PATENT SPECIFICATION

(11) **EP 2 542 572 B1**
(45) Date of publication and mention of the grant of the patent: **06.05.2015**
(21) Application number: 11711151.8
(22) Date of filing: 02.03.2011
(51) Int. Cl.: C07K 14/525

(54) **MULTIMERIC FORMS OF THERAPEUTIC PROTEINS AND USES THEREOF**
MULTIMERE FORMEN VON THERAPEUTISCHEN PROTEINEN UND IHRE VERWENDUNG
FORMES MULTIMÈRES DE PROTÉINES THÉRAPEUTIQUES ET LEURS APPLICATIONS

(30) Priority: 02.03.2010 US 309487 P
(43) Date of publication of application: 09.01.2013
(73) Proprietor: Protalix Ltd., 2010000 Carmiel (IL)
(72) Inventor: RUDERFER, Ilya, 21811 Carmiel (IL); SHILOVITTZKY, Orit, 34672 Haifa (IL); SHULMAN, Avidor, 2017500 Doar-Na Misgav (IL); SHAALTIEL, Yoseph, 3657600 Timrat (IL); BEN-MOSHE, Tehila, 20181 Doar-Na Misgav (IL); SHEKHTER, Talia, 53452 Givataim (IL); AZULAY, Yaniv, 24315 Akko (IL)
(74) Representative: Gervasi, Gemma
(86) International application number: PCT/IL2011/000211
(87) International publication number: WO 2011/107992

(56) References cited:
- WO-A1-01/25277
- WO-A1-95/01994
- WO-A2-93/18148
- WO-A2-03/042244
- WO-A2-2009/024977
- US-A1- 2005 180 948

## Description

### FIELD AND BACKGROUND OF THE INVENTION

The present invention, in some embodiments thereof, relates to novel multimeric protein structures and, more particularly, but not exclusively, to multimeric protein structures of exemplary therapeutic proteins and to uses thereof in treating diseases or disorders.

Many proteins occur naturally in a multimeric form, comprising more than monomers, each monomer comprising one polypeptide chain. In addition to the tertiary structure of each monomer, multimeric proteins possess a quaternary structure, which is the arrangement into which the monomers assemble. Changes in quaternary structure can occur through conformational changes within individual monomers or through reorientation of the monomers relative to each other. It is through such changes, which underlie cooperativity and allostery in multimeric enzymes, that the physiological activity of many proteins is regulated.

In some cases, a quaternary structure is achieved or maintained through covalent disulfide bonds. In other examples the quaternary structure is only maintained through non-covalent bonds based mainly on ionic, hydrophobic and van-der-Waals interactions.

In some multimeric proteins, the biological activity of the protein is highly dependent upon the quaternary structure of the multimeric form. Thus, a monomeric form of the protein exhibits considerably reduced activity and/or less stability in comparison with the multimeric form, and may even be entirely inactive.

The following lists exemplary such proteins:
Luteinizing hormones are structured as heterodimers, composed of two subunits, α and β. Only the heterodimeric protein exhibits a biological activity, and this activity is lost once the dimeric structure is lost or the subunits are not dimerized properly.

Follicle-stimulating hormone (FSH), thyroid-stimulating hormone (TSH) and human chorionic gonadotropin (hCG) are heterodimeric hormones having a similar structure to that or luteinizing hormones. Luteinizing hormone, FSH, TSH and hCG have the same α subunits, but comprise different β subunits.

Antibodies, also known as "immunoglobins", are also proteins with complex quaternary structures, being composed of two chains, the light chain and the heavy chain. An immunoglobin domain comprises two light chains and two heavy chains which are covalently bound to one another by disulfide bonds, and an immunoglobin protein may comprise one domain (as in IgD, IgE and IgG), two domains (as in IgA) or five domains (as in IgM).

The soluble cytokine TNF-α (sTNF) is a non-covalent homotrimeric protein. At concentrations below the nanomolar range, the subunits of the trimer tend to dissociate, and the protein thereby loses its biological activity.

TNF-α receptor (TNFR) is activated when 2 or 3 TNFR protein molecules bind to a single trimeric TNF-α protein, inducing formation of a multimeric form of TNFR.

Etanercept is a fusion protein of TNFR with an Fc region of an immunoglobin (IgG) heavy chain. Two identical polypeptide chains, each being a fusion protein, are covalently bound by disulfide bonds, resulting in a structure similar to that of an IgG protein, which comprises a pair of heavy chains. This dimeric protein functions as a TNFR dimer, which is more effective at binding TNF-α than is monomeric TNFR. However, etanercept does not engage directly in cell signaling, but is rather used as a scavenger of TNF-α, in the treatment of rheumatoid arthritis.

Abatacept is another homodimeric fusion protein, being composed of two homologous cytotoxic T-lymphocyte-associated antigen-4 (CTLA-4) receptor extracellular domain polypeptide chains (two identical active portions of CTLA-4) fused with a modified Fc region of a human IgG heavy chain. Native CTLA-4 is a receptor for CD86, and is most effective at binding CD86 when in a dimeric form linked by disulfide bonds. Abatacept is designed to mimic the active dimeric form by comprising two CTLA4-immunoglobin linked by a disulfide bond and non-covalent interactions. By binding CD86, abatacept inhibits immune responses, and is used in the treatment of rheumatoid arthritis.

VEGF (vascular endothelial growth factor) and PDGF (platelet-derived growth factor) are homodimeric proteins which act by binding to receptor tyrosine kinases (VEGF receptor and PDGF receptor, respectively), causing the receptors to dimerize. The receptors exert a tyrosine kinase activity in their dimeric form.

Urate oxidase is a homotetrameric enzyme, typically localized mainly in the liver, which catalyzes the oxidation of uric acid. Urate oxidase is present in many animals, but is not expressed in humans and in some primates. Rasburicase is a recombinant version of urate oxidase, which is used for treating acute hyperuricemia in patients receiving chemotherapy. Pegloticase is a PEGylated recombinant urate oxidase used to treat gout. As a result of PEGylation, pegloticase exhibits a longer elimination half life (about 10-12 days) than does rasburicase.

Interleukin-17 (IL-17) is a cytokine which is a homodimeric glycoprotein linked by disulfide bonds. IL-17 induces and mediates pro-inflammatory responses, including allergic responses, and induces the production of a variety of prostaglandins, cytokines and chemokines, including TNF-α.

### SUMMARY OF THE INVENTION

According to an aspect of some embodiments of the present invention there is provided a multimeric protein structure comprising at least two monomers of a therapeutic protein, the monomers being covalently linked to one another via a non-peptidic linking moiety, the multimeric protein structure featuring a characteristic selected from the group consisting of:
(a) a biological activity upon subjecting the multimeric protein structure to physiological conditions for one day, which is at least 10 % higher than an activity of a native form of the therapeutic protein upon subjecting the native form of the protein to physiological conditions for one day;
(b) a biological activity which decreases upon subjecting the multimeric protein structure to physiological conditions for one day by a percentage which is at least 10 % less than the percentage by which an activity of the native form of the therapeutic protein decreases upon subjecting the native form of the protein to physiological conditions for one day; and
(c) a circulating half-life in a physiological system which is higher by at least 20 % than a circulating half-life of the native form of the protein,
   the therapeutic protein being selected from the group consisting of a TNF-α and fragments thereof wherein said at least one non-peptidic linking moiety has a general formula:

   -X₁-(CR₁R₂-CR₃R₄-Y)n-X₂-
wherein each of X₁ and X₂ is a functional group that forms a covalent bond with at least one of said monomers;
Y is O, S or NR₅;
n is 3, 4, 5, 6, 7 or 8; and
each of R₁, R₂, R₃, R₄ and R₅ is independently selected from the group consisting of hydrogen, alkyl, cycloalkyl, alkenyl, alkynyl, alkoxy, hydroxy, oxo, thiol and thioalkoxy.

According to an aspect of some embodiments of the present invention there is provided a multimeric protein structure comprising at least two monomers of a therapeutic protein, the monomers being covalently linked to one another via a bifunctional non-peptidic linking moiety comprising a poly(alkylene glycol), the linking moiety being no more than 30 atoms long, the therapeutic protein being selected from the group consisting of a TNF-α and fragments thereof.

According to an aspect of some embodiments of the present invention there is provided a pharmaceutical composition comprising a multimeric protein structure described herein and a pharmaceutically acceptable carrier.

According to an aspect of some embodiments of the present invention there is provided a process of preparing a multimeric protein structure described herein, the process comprising reacting the therapeutic protein with a cross-linking agent which comprises the linking moiety and at least two reactive groups.

According to some embodiments of the invention, the therapeutic protein is a TNF-α.

According to some embodiments of the invention, the biological activity of the multimeric protein structure remains substantially unchanged upon subjecting the multimeric protein structure to physiological conditions for 8 hours.

According to some embodiments of the invention, the multimeric protein structure features a characteristic selected from the group consisting of:
(a) a biological activity upon subjecting the multimeric protein structure to physiological conditions for one day, which is at least 10 % higher than an activity of a native form of the therapeutic protein upon subjecting the native form of the protein to physiological conditions for one day;
(b) a biological activity which decreases upon subjecting the multimeric protein structure to physiological conditions for one day by a percentage which is at least 10 % less than the percentage by which an activity of the native form of the therapeutic protein decreases upon subjecting the native form of the protein to physiological conditions for one day; and
(c) a circulating half-life in a physiological system which is higher by at least 20 % than a circulating half-life of the native form of the protein,
   the biological activity being binding to a TNF-α receptor.

According to some embodiments of the invention, the therapeutic protein is a human protein.

According to some embodiments of the invention, the poly(alkylene glycol) comprises two functional groups, each functional group forming a covalent bond with one of the monomers.

According to some embodiments of the invention, the two functional groups are terminal groups of the poly(alkylene glycol).

According to some embodiments of the invention, the at least one linking moiety has a general formula:

-X₁-(CR₁R₂-CR₃R₄-Y)n-X₂-

wherein each of X₁ and X₂ is a functional group that forms a covalent bond with at least one of the monomers;
Y is O, S or NR₅;
n is 3, 4, 5, 6, 7, 8 or 9; and
each of R₁, R₂, R₃, R₄ and R₅ is independently selected from the group consisting of hydrogen, alkyl, cycloalkyl, alkenyl, alkynyl, alkoxy, hydroxy, oxo, thiol and thioalkoxy.

According to some embodiments of the invention, at least one of the functional groups forms an amide bond with a monomer of the therapeutic protein.

According to some embodiments of the invention, n is an integer from 3 to 7.

According to some embodiments of the invention, the multimeric protein structure is for use as a medicament.

According to some embodiments of the invention, the medicament is for treating a disease or disorder treatable by the therapeutic protein.

According to some embodiments of the invention, the multimeric protein structure is for use in treating a disease or disorder treatable by the therapeutic protein.

According to some embodiments of the invention, the process described herein comprises reacting the therapeutic protein with the cross-linking agent under conditions in which the therapeutic protein is in a multimeric form.

According to some embodiments of the invention, the reactive groups comprise a leaving group.

According to some embodiments of the invention, the reactive group reacts with an amine group to form an amide bond.

According to some embodiments of the invention, each of the reactive groups is capable of forming a covalent bond between the linking moiety and at least one of the monomers.

According to some embodiments of the invention, a molar ratio of the cross-linking agent to monomers of the therapeutic protein is in a range of from 5:1 to 500:1.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
FIG. 1 presents an image of an SDS-polyacrylamide (15 %) electrophoresis gel showing TNF-α (tumor necrosis factor-α) which was reacted with bis-NHS-PEG₅, bis-NHS-PEG₉, and bis-NHS-PEG₂₁, as well as molecular weight markers (M.W.) and non-reacted TNF-α;
FIG. 2 is a graph showing the viability of L-929 fibroblasts following treatment with various concentrations of TNF-α or TNF-α cross-linked with bis-NHS-PEG₅ (cross 5), bis-NHS-PEG₉ (cross-9) or bis-NHS-PEG₂₁ (cross 21), relative to viability of untreated cells; and
FIG. 3 is a bar graph showing the binding of TNF-α (red) and TNF-α cross-linked with bis-NHS-PEG₅ (green) to etanercept after incubation in phosphate buffer for 2 hours (2hr), 8 hours (8hr) or overnight (ON), at a temperature of 37 °C (37) or 4 °C (4.00); results are normalized to those obtained after overnight incubation at 4 °C (on left).

### DESCRIPTION OF SPECIFIC EMBODIMENTS OF THE INVENTION

The present invention, in some embodiments thereof, relates to novel multimeric protein structures and, more particularly, but not exclusively, to multimeric protein structures of therapeutic proteins and to uses thereof in treating diseases or disorders.

Many diseases and disorders are associated with a deficiency of a protein and/or an excess of a protein or metabolite. Administration of a protein may be effective by overcoming a deficiency of a protein, and/or by binding to or otherwise inactivating a protein or metabolite present in excess. However, administration of a protein does not necessarily result in a considerable and/or persistent activity of the protein *in vivo.*

As shown herein, the activity of a multimeric protein (e.g., TNF-α) may be compromised under physiological conditions, thereby reducing its potentially therapeutic effects.

Motivated by a need to solve the compromised activity of therapeutic proteins such as TNF-α, the present inventors have searched for stabilized forms of therapeutic proteins. More specifically, the present inventors have envisioned that a stabilized form of a therapeutic protein would exhibit longer lasting activity in general, and longer lasting activity in serum in particular. The present inventors have thus designed and successfully prepared and practiced stabilized forms of exemplary native therapeutic protein and have indeed shown that such stabilized forms of these proteins exhibit an improved performance, at least in terms of a longer lasting activity under physiological conditions.

The present inventors have demonstrated a formation of stabilized forms of exemplary therapeutic proteins which exhibit an improved performance by means of cross-linking the native protein, via formation of new covalent linkage between monomeric proteins.

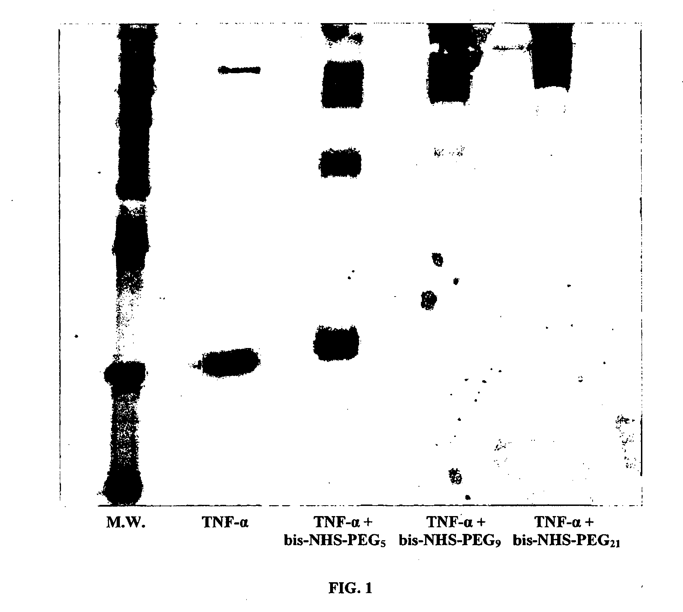

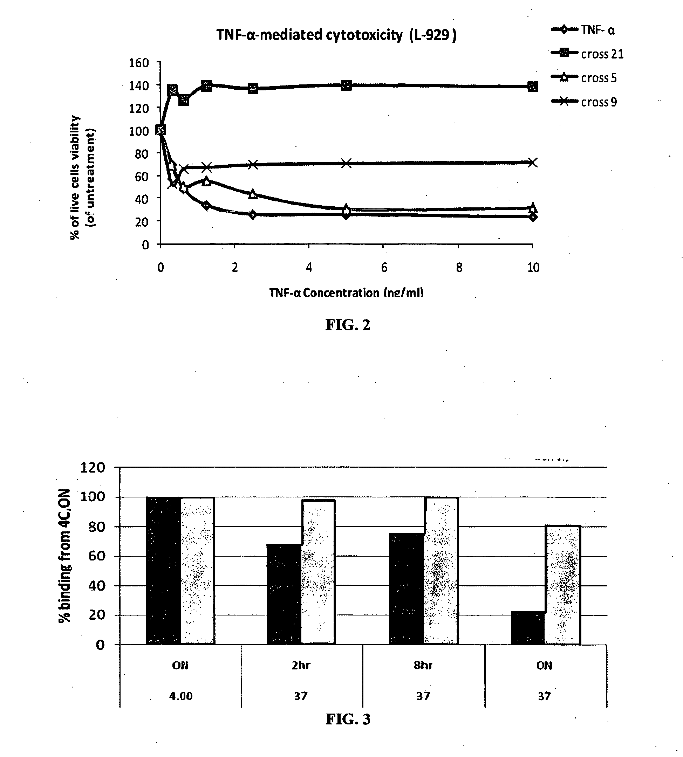

Referring now to the drawings, Figure 1 shows that TNF-α was covalently cross-linked by reaction with exemplary cross-linking reagents. Figure 2 shows that cross-linking by relatively short cross-linkers did not interfere with the biological activity of TNF-α. Figure 3 shows that covalently cross-linked TNF-α exhibits a longer lasting activity under physiological conditions (pH 7.4, 37 °C) than does native TNF-α.

The above results indicate that covalently cross-linking a therapeutic protein results in a multimer with improved stability, which allows for a longer lasting and therefore more effective activity under physiological conditions.

Hence, according to an aspect of some embodiments of the present invention there is provided a multimeric protein structure comprising at least two monomers of a therapeutic protein, as defined herein, the monomers being covalently linked to one another via a linking moiety, the multimeric protein structure featuring a stability higher than that of a corresponding native therapeutic protein, as described in detail below.

TNF-α exhibits an immunostimulating effect, and may be effective at providing a stronger immune response against a harmful target (e.g., cancer cells).

Exemplary TNF-α has SEQ ID NO: 1.

The multimeric protein structure may be, for example, a dimer, a trimer, a tetramer, a pentamer, a hexamer, a heptamer or an octamer. In some embodiments, the multimeric protein structure contains the same number of monomers as a native form of the therapeutic protein. Alternatively, the multimeric structure may contain a different number of monomers (e.g., more monomers) than the native form, for example, twice, three times, or four times the number of monomers present in the native form.

The indicated therapeutic protein may optionally be a fragment of a protein, for example, a soluble fragment of a membrane protein. Optionally, the fragment is characterized by a therapeutically effective activity (e.g., the protein fragment exhibits an activity of the whole protein). For example, a binding activity exhibited by a therapeutic protein may also be exhibited by a fragment of the protein.

Optionally, the indicated therapeutic protein is a human protein. Human proteins are typically more suitable for administration to a human subject than are other proteins. For example, use of a human protein may minimize or even entirely avoid an immune response against the protein in a human. In addition, a human protein may better interact with proteins, cells and/or tissues which are present in a human body.

Herein, a "human protein" refers to a protein comprising an amino acid sequence substantially identical (e.g., as described herein) to an amino acid sequence of a protein which naturally occurs in humans.

Herein, the term "native" with respect to a protein encompasses proteins comprising an amino acid sequence substantially identical (i.e., at least 95 % homology, optionally at least 99 % homology, and optionally 100 %) to an amino acid sequence of a naturally occurring protein. A native protein may be a protein isolated from a natural source, or a recombinantly produced protein (e.g., derived from mammalian cells, plant cells, bacterial cells, fungal cells). A native form of a protein further comprises a secondary and tertiary structure (e.g., folding) substantially identical to that of a naturally occurring protein.

The term "native", when used in reference to a quaternary structure of a protein (e.g., a multimeric protein), further comprises a quaternary structure substantially identical to that of a naturally occurring protein.

Herein, the phrase "naturally occurring protein" refers to a protein in a form which occurs in nature (e.g., in an organism), with respect to the protein's amino acid sequence, secondary structure and tertiary structure, as well as the protein's quaternary structure if the protein is in a multimeric form.

Post-translational modifications (e.g., glycosylation) of naturally occurring proteins (e.g., in an organism which expresses the naturally occurring protein) may be present, absent or modified in the native form of a protein described herein. A native form of a protein (e.g., a recombinantly produced protein) may optionally comprise different post-translational modifications than those of the naturally occurring protein, provided that the native form of the protein retains a substantially similar amino acid sequence and structure as the naturally occurring protein, as described above.

Herein, the native form of a protein may refer to a monomeric structure, a dimeric structure and/or a multimeric structure. For example, a multimeric protein can be described as a native form of a multimeric protein, and a monomeric polypeptide in a multimeric protein can be described as a native form of the monomeric polypeptide.

A multimeric native form of a protein typically comprises non-covalent bonds between the monomeric polypeptides of the multimer, such as hydrophobic interactions, hydrogen bonds, Van der Waals interactions and/or ionic bonds.

In some embodiments, a multimeric native form of a protein further comprises covalent bonds, generally disulfide bonds, between the monomeric polypeptides therein.

The multimeric protein structures described herein comprise covalent bonds which link the monomeric polypeptides therein, and which is absent from the native form of the protein.

The linking moiety which links the monomers is a moiety which is not present in a native form of the therapeutic protein (e.g., a synthetic linking moiety).

Thus, for example, the linking moiety is optionally a moiety which is covalently attached to a side chain, an N-terminus or a C-terminus, or a moiety related to post-translational modifications (e.g., a saccharide moiety) of a protein monomer, as well as to a side chain, an N-terminus or a C-terminus, or a moiety related to post-translational modifications (e.g., a saccharide moiety) of another protein monomer. Exemplary such linking moieties are described in detail hereinunder.

Optionally, the linking moiety is devoid of a disulfide bond. However, a linking moiety which includes a disulfide bond at a position such that the disulfide bond is not essential for forming a link between monomers (e.g., cleavage of the disulfide bond does not cleave the link between the monomers) is within the scope of this embodiment of the invention. A potential advantage of linking moiety devoid of a disulfide bond is that it is not susceptible to cleavage by mildly reducing conditions, as are disulfide bonds.

The linking moiety is a non-peptidic moiety (e.g., the linking moiety does not consist of an amide bond, an amino acid, a dipeptide, a tripeptide, an oligopeptide or a polypeptide). A potential advantage of a linking moiety which is a non-peptidic moiety is that it is not susceptible to cleavage by proteases and peptidases (e.g., such as are present *in vivo*).

The linking moiety is also referred to herein as a cross-linking moiety. The linking of protein monomers by a linking moiety is referred to herein as "cross-linking".

The cross-linking moiety is a bridging moiety (composed of a chain of chemical groups).

A "bridging moiety" refers to a multifunctional moiety (e.g., biradical, triradical, etc.) that is attached to side chains, moieties related to post-translational modifications (e.g., saccharide moieties) and/or termini (i.e., N-termini, C-termini) of two or more of the monomers.

As exemplified herein in the Examples section, relatively short linking moieties (e.g., PEG₅) may be more effective than longer linking moieties (e.g., PEG₂₁) at cross-linking between different protein monomers while preserving a biological activity of the protein.

Hence, according to some embodiments, the linking moiety is no more than 30 atoms long, optionally no more than 20 atoms long, and optionally no more than 10 atoms long.

Herein, the length of a linking moiety (when expressed as a number of atoms) refers to length of the backbone of the linking moiety, i.e., the number atoms forming a linear chain between residues of each of two monomers linked via the linking moiety.

Optionally, the linking moiety is below a certain size, so as to avoid an unnecessarily excessive part of the linking moiety in the formed cross-linked protein, which may interfere with the function of the protein, and/or so as to avoid complications and/or inefficiency in a synthesis of the linking moiety. In addition, in some embodiments, a large linking moiety may be less effective at cross-linking between different monomers.

Hence, according to some embodiments, each linking moiety is characterized by a molecular weight of less than 5 KDa, optionally less than 3 KDa, optionally less than 2 KDa, optionally less than 1 KDa, and optionally less than 0.5 KDa.

In order to facilitate cross-linking, the linking moiety is optionally substantially flexible, wherein the bonds in the backbone of the linking moiety are mostly rotationally free, for example, single bonds which are not coupled to a double bond (e.g., unlike an amide bond) and wherein rotation is not sterically hindered. Optionally, at least 70 %, optionally at least 80 %, and optionally at least 90 % (e.g., 100 %) of the bonds in the backbone of the linking moiety are rotationally free.

In some embodiments, the linking moiety comprises a poly(alkylene glycol) chain.

The phrase "poly(alkylene glycol)", as used herein, encompasses a family of polyether polymers which share the following general formula: -O-[(CH₂)ₘ-O-]ₙ-, wherein m represents the number of methylene groups present in each alkylene glycol unit, and n represents the number of repeating units, and therefore represents the size or length of the polymer. For example, when m = 2, the polymer is referred to as a polyethylene glycol, and when m = 3, the polymer is referred to as a polypropylene glycol.

In some embodiments, m is an integer greater than 1 (e.g., m = 2, 3, 4, etc.).

Optionally, m varies among the units of the poly(alkylene glycol) chain. For example, a poly(alkylene glycol) chain may comprise both ethylene glycol (m=2) and propylene glycol (m=3) units linked together.

The poly(alkylene glycol) optionally comprises at least two functional groups (e.g., as described herein), each functional group forming a covalent bond with one of the protein monomers. The functional groups are optionally terminal groups of the poly(alkylene glycol), such that the entire length of the poly(alkylene glycol) lies between the two functional groups.

The phrase "poly(alkylene glycol)" also encompasses analogs thereof, in which the oxygen atom is replaced by another heteroatom such as, for example, S, -NH- and the like. This term further encompasses derivatives of the above, in which one or more of the methylene groups composing the polymer are substituted. Exemplary substituents on the methylene groups include, but are not limited to, alkyl, cycloalkyl, alkenyl, alkynyl, alkoxy, hydroxy, oxo, thiol and thioalkoxy, and the like.

The phrase "alkylene glycol unit", as used herein, encompasses a -(CH₂)ₘ-O-group or an analog thereof, as described hereinabove, which forms the backbone chain of the poly(alkylene glycol), wherein the (CH₂)ₘ (or analog thereof) is bound to a heteroatom belonging to another alkylene glycol unit or to a protein monomer moiety (in cases of a terminal unit), and the O (or heteroatom analog thereof) is bound to the

(CH₂)ₘ (or analog thereof) of another alkylene glycol unit, or to a functional group which forms a bond with a protein monomer.

An alkylene glycol unit may be branched, such that it is linked to 3 or more neighboring alkylene glycol units, wherein each of the 3 or more neighboring alkylene glycol units are part of a poly(alkylene glycol) chain. Such a branched alkylene glycol unit is linked via the heteroatom thereof to one neighboring alkylene glycol unit, and heteroatoms of the remaining neighboring alkylene glycol units are each linked to a carbon atom of the branched alkylene glycol unit. In addition, a heteroatom (e.g., nitrogen) may bind more than one carbon atom of an alkylene glycol unit of which it is part, thereby forming a branched alkylene glycol unit (e.g., [(-CH₂)ₘ]₂N- and the like).

In exemplary embodiments, at least 50 % of alkylene glycol units are identical, e.g., they comprise the same heteroatoms and the same m values as one another. Optionally, at least 70 %, optionally at least 90 %, and optionally 100 % of the alkylene glycol units are identical. In exemplary embodiments, the heteroatoms bound to the identical alkylene glycol units are oxygen atoms. In further exemplary embodiments, m is 2 for the identical units.

In one embodiment, the linker is a single, straight chain linker, preferably being polyethylene glycol (PEG).

As used herein, the term "poly(ethylene glycol)" describes a poly(alkylene glycol), as defined hereinabove, wherein at least 50 %, at least 70 %, at least 90 %, and preferably 100 %, of the alkylene glycol units are -CH₂CH₂-O-. Similarly, the phrase "ethylene glycol units" is defined herein as units of -CH₂CH₂O-.

According to optional embodiments, the linking moiety comprises a poly(ethylene glycol) or analog thereof, having a general formula:

-X₁-(CR₁R₂-CR₃R₄-Y)ₙ-X₂-

wherein each of X₁ and X₂ is a functional group (e.g., as described herein) that forms a covalent bond with at least one protein monomer;
Y is O, S or NR₅ (optionally O);
n is 3,4, 5, 6, 7 or 8; and
each of R₁, R₂, R₃, R₄, and R₅ is independently selected from the group consisting of hydrogen, alkyl, cycloalkyl, alkenyl, alkynyl, alkoxy, hydroxy, oxo, thiol and thioalkoxy.

In some embodiments, n is at least 4.

In some embodiments n is from 3 to 7. Thus, in some embodiments, n can be 3, 4, 5, 6 or 7.

The poly(ethylene glycol) or analog thereof may optionally comprise a copolymer, for example, wherein the CR₁R₂-CR₃R₄-Y units in the above formula are not all identical to one another.

In some embodiments, at least 50 % of CR₁R₂-CR₃R₄-Y units are identical. Optionally, at least 70 %, optionally at least 90 %, and optionally 100 % of the CR₁R₂-CR₃R₄-Y units are identical.

Optionally, the linking moiety is branched, for example, such that for one or more CR₁R₂-CR₃R₄-Y units in the above formula, at least of one of R₁, R₂, R₃, R₄, and R₅ is -(CR₁R₂-CR₃R₄-Y)ₚ-X₃-, wherein R₁-R₄ and Y are as defined hereinabove, p is an integer as defined herein for n (e.g., from 1 to 200), and X₃ is as defined herein for X₁ and X₂.

The functional groups form a covalent bond with at least one of the monomers such as, but not limited to, an amide bond, an amine bond, an ester bond, and/or an ether bond.

For example, the functional group may optionally comprise a carbonyl group which forms an amide bond with a nitrogen atom in a polypeptide (e.g., in a lysine residue or N-terminus), or an ester bond with an oxygen atom in a polypeptide (e.g., in a serine, threonine or tyrosine residue).

Alternatively or additionally, the functional group may optionally comprise a heteroatom (e.g., N, S, O) which forms an amide bond, ester bond or thioester bond with a carbonyl group in a polypeptide (e.g., in a glutamate or aspartate residue or in a C-terminus).

Alternative or additionally, the functional group may comprise an alkyl or aryl group attached to a polypeptide (e.g., to a heteroatom in the polypeptide).

Alternatively or additionally, the functional group may optionally comprise a nitrogen atom which forms an amine bond with an alkyl group in a monomer, or the monomer may optionally comprise a nitrogen atom which forms an amine bond with an alkyl group in the functional group. Such an amine bond may be formed by reductive amination (e.g., as described hereinbelow).

In some embodiments, at least one of the functional groups forms an amide bond with a polypeptide (e.g., with a lysine residue therein).

The functional groups may be identical to one another or different.

In some embodiments, at least one of the functional groups is attached to one functionality of a protein monomer (e.g., an amine group of a lysine residue or N-terminus), and at least one of the functional groups is attached to a different functionality of a protein monomer (e.g., a thiol group of a cysteine residue).

In some embodiments, the therapeutic protein is TNF-α.

In some embodiments, the multimeric protein structure comprises three TNF-α monomers (i.e., the protein structure is a trimer), the monomers being cross-linked by a polyethylene glycol linking moiety described herein. In exemplary embodiments, the linking moiety is a PEG₅ linking moiety, as described herein.

As exemplified herein, the multimeric protein structure described herein exhibits a high stability under physiological conditions.

As used herein, "stable activity" means that the activity of the protein structure is long-lasting when the protein structure is exposed to conditions such as are described herein.

As used herein, the phrase "physiological conditions" refers to an aqueous solution (e.g., phosphate buffer) with a pH of 7.4, at a temperature of 37 °C.

Without being bound by any particular theory, it is believed that enhanced stability under physiological conditions is advantageous because a stable therapeutic protein in a body (e.g., in serum) can exert a therapeutically beneficial activity for a longer period of time, resulting in a more significant effect *in vivo.*

According to optional embodiments, the high stability of the multimeric protein structure under physiological conditions is such that the multimeric protein structure exhibits, upon being subjected to physiological conditions for one day, an activity which is at least 10 % higher, optionally 20 % higher, optionally 50 % higher, and optionally 100 % higher, than an activity of the native form of the therapeutic protein upon subjecting the native form of the therapeutic protein to the physiological conditions for one day.

Alternatively or additionally, the high stability of the multimeric protein structure under physiological conditions is such that an activity of the multimeric protein structure decreases more slowly in than a corresponding activity of the native form of the therapeutic protein. Optionally, the multimeric protein structure exhibits an activity which decreases upon subjecting the protein structure to physiological conditions for one day by a percentage which is at least 10 % less, optionally 20 % less, optionally 50 % less, and optionally 80 % less, than the percentage by which a corresponding activity of the native therapeutic protein decreases upon subjecting the native therapeutic protein to physiological conditions for one day.

It is to be understood that herein, a decrease which is "10 % less" than a decrease of 50 % refers to a decrease of 45 % (45 being 10 % less than 50), and not to a decrease of 40 % (50 % - 10%).

Alternatively or additionally, the high stability of the multimeric protein structure under physiological conditions is such that an activity of the multimeric protein structure remains substantially unchanged upon subjecting the multimeric protein structure to physiological conditions for 8 hours, and optionally for 24 hours, for 2 days, for 4 days, and even 1 week.

As used herein, the phrase "substantially unchanged" refers to a level (e.g., of activity) which remains in a range of from 50 % to 150 % of the initial level, and optionally a level which remains at least 60 %, optionally at least 70 %, optionally at least 80 %, and optionally at least 90 % of the initial level.

The activity described herein is a biological activity. A biological activity may refer, for example, to a catalytic activity (e.g., wherein the protein is an enzyme) or a binding activity (e.g., wherein the protein is a receptor and/or ligand), such as binding of an ion, a metal, a protein, or any other biomolecule.

In some embodiments, a catalytic activity is characterized by a rate of catalysis at saturation (i.e., a *Vₘₐₓ* value).

In some embodiments, a binding activity is characterized by a binding capacity.

Optionally, the activity is a therapeutic activity (e.g., an enzymatic activity having a therapeutic effect).

Techniques for determining an activity of a protein will be known to a skilled person.

For example, a binding capacity may be determined by binding the protein (i.e., the native form or a multimeric protein structure described herein) to an excess of a compound (e.g., a receptor) which specifically binds to the protein, and then quantitatively measuring the amount of bound protein (e.g., by ELISA, as described herein).

In another typical example, a protein (i.e., the native form or a multimeric protein structure described herein) is contacted with a compound recognized in the art as interacting with the protein (e.g., a substrate of an enzyme, a ligand of a receptor), and the degree of activity is then determined quantitatively. Many compounds (e.g., substrates, ligands) which allow for particularly convenient detection of protein activity are known in the art, for example, compounds which are readily detectable (e.g., chromophores, fluorophores, radiolabeled compounds) or which form a readily detectable compound when acted upon (e.g., by an enzyme).

When comparing an activity of a multimeric protein structure described herein with an activity of a native form of a therapeutic protein, the native form preferably comprises monomers substantially identical (e.g., with respect to amino acid sequence and glycosylation pattern) to the monomers of the multimeric structure.

According to some embodiments, the multimeric protein structure is characterized by a circulating half-life in a physiological system (e.g., blood, serum and/or plasma of a human or laboratory animal) which is higher (e.g., at least 20 %, at least 50 % higher, at least 100 % higher, at least 400 % higher, at least 900 % higher) than a circulating half-life of native therapeutic protein.

An increased circulating half-life may optionally be associated with a higher stability under physiological conditions (e.g, as described herein), a higher *in vivo* stability (e.g, resistance to metabolism) and/or with other factors (e.g., reduced renal clearance).

Circulating half-lives can be determined by taking samples (e.g., blood samples, tissue samples) from physiological systems (e.g., humans, laboratory animals) at various intervals, and determining a level of the protein in the sample, using techniques known in the art.

Optionally, the half-life is calculated as a terminal half-life, wherein half-life is the time required for a concentration (e.g., a blood concentration) to decrease by 50 % after pseudo-equilibrium of distribution has been reached. The terminal half-life may be calculated from a terminal linear portion of a time vs. log concentration, by linear regression of time vs. log concentration (see, for example, Toutain & Bousquet-Melou [J Vet Pharmacol Ther 2004, 27:427-39]). Thus, the terminal half-life is a measure of the decrease in drug plasma concentration due to drug elimination and not of decreases due to other reasons, and is not necessarily the time necessary for the amount of the administered drug to fall by one half.

Determining a level of the multimeric protein structure or the native form of the therapeutic protein may comprise detecting the physical presence of the protein (e.g., via an antibody against the protein, optionally via ELISA) and/or detecting a level of an activity of the protein (e.g., as described herein).

As exemplified in the Examples section herein, a multimeric protein structure described herein may be conveniently prepared by reacting a therapeutic protein with a cross-linking agent.

Hence, according to another aspect of embodiments of the invention, there is provided a process of preparing a multimeric protein structure described herein. The process comprises reacting a therapeutic protein (e.g., as described herein), so as to introduce at least one linking moiety which covalently links at least two monomers of the therapeutic protein.

In some embodiments, the linking moiety is introduced by reacting the therapeutic protein with a cross-linking agent which comprises the linking moiety (e.g., as described herein) and at least two reactive groups.

Optionally, the therapeutic protein is reacted under conditions in which the therapeutic protein is in a multimeric form.

In some embodiments, the cross-linking agent is reacted with the therapeutic protein at a molar ratio in a range of from 5:1 to 500:1 (cross-linking agent: protein monomer), optionally in a range of from 20:1 to 400:1, and optionally in a range of from 50:1 to 300:1 (e.g., about 100:1).

According to some embodiments, the molar ratio is 25:1, 30:1, 40:1, 50:1, 60:1, 70:1, 75:1, 80:1, 90:1, 100:1, 110:1, 120:1, 130:1, 140:1, 150:1, 160:1, 170:1, 180:1, 190:1, 200:1, 250:1, or 300:1, with any values between the above-indicated values being also contemplated.

The process optionally further comprises purifying the cross-linked protein, for example, removing excess cross-linking agent. Common purification methods may be used, such as dialysis and/or ultra-filtration using appropriate cut-off membranes and/or additional chromatographic steps, including size exclusion chromatography, ion exchange chromatography, affinity chromatography, hydrophobic interaction chromatography, and the like.

The reactive group is selected suitable for undergoing a chemical reaction that leads to a bond formation with a complementary functionality in the protein monomer. Optionally, each reactive group is capable of forming a covalent bond between the linking moiety described herein and at least one protein monomer (e.g., so as to form a functional group bound to the protein monomer, as described herein).

The reactive groups of a cross-linking agent may be identical to one another or different.

As used herein, the phrase "reactive group" describes a chemical group that is capable of undergoing a chemical reaction that typically leads to a bond formation. The bond, according to the present embodiments, is preferably a covalent bond (e.g., for each of the reactive groups). Chemical reactions that lead to a bond formation include, for example, nucleophilic and electrophilic substitutions, nucleophilic and electrophilic addition reactions, alkylations, addition-elimination reactions, cycloaddition reactions, rearrangement reactions and any other known organic reactions that involve a functional group, as well as combinations thereof.

The reactive group may optionally comprise a non-reactive portion (e.g., an alkyl) which may serve, for example, to attach a reactive portion of the reactive group to a linking moiety (e.g., poly(alkylene glycol) or analog thereof) described herein.

The reactive group is preferably selected so as to enable its conjugation to the therapeutic protein. Exemplary reactive groups include, but are not limited to, carboxylate (e.g., -CO₂H), thiol (-SH), amine (-NH₂), halo, azide (-N₃), isocyanate (-NCO), isothiocyanate (-N=C=S), hydroxy (-OH), carbonyl (e.g., aldehyde), maleimide, sulfate, phosphate, sulfonyl (e.g. mesyl, tosyl), etc. as well as activated groups, such as *N*-hydroxysuccinimide (NHS) (e.g. NHS esters), sulfo-*N*-hydroxysuccinimide, anhydride, acyl halide (-C(=O)-halogen) etc.

In some embodiments, the reactive group comprises a leaving group, such as a leaving group susceptible to nucleophilic substitution (e.g., halo, sulfate, phosphate, carboxylate, N-hydroxysuccinimide).

Optionally, the reactive group may be in an activated form thereof.

As used herein, the phrase "activated form" describes a derivative of a chemical group (e.g., a reactive group) which is more reactive than the chemical group, and which is thus readily capable of undergoing a chemical reaction that leads to a bond formation. The activated form may comprise a particularly suitable leaving group, thereby facilitating substitution reactions. For example, a -C(=O)-NHS group (N-hydroxysuccinimide ester, or -C(=O)-O-succinimide) is a well-known activated form of -C(=O)OH, as NHS (N-hydroxysuccinimide) can be reacted with a -C(=O)OH to form -C(=O)-NHS, which readily reacts to form products characteristic of reactions involving -C(=O)OH groups, such as amides and esters.

The reactive group can be attached to the rest of the linking moiety (e.g., a poly(alkylene glycol) or analog thereof) via different groups, atoms or bonds. These may include an ether bond [e.g., -O-alkyl-], an ester bond [e.g., -O-C(=O)-alkyl-], a carbamate [e.g., O-C(=O)-NH-alkyl-], etc. Thus, a variety of terminal groups can be employed.

The following are non-limiting examples of the different groups that may constitute a reactive group as described herein: -CH₂CO₂H, -CH₂CH₂CO₂H, -CH₂CH₂SH, -CH₂CH₂NH₂, -CH₂CH₂N₃, -CH₂CH₂NCO, -CH₂-C(=O)-NHS, -CH₂CH₂-C(=O)-NHS, -C(=O)-CH₂-C(=O)-NHS, -CH₂CH₂-NHC(=O)CH₂CH₂-maleimide, etc.

The number of methylene groups in each of the above reactive groups is merely exemplary, and may be varied.

The reactive group may also comprise the heteroatom at the end of a poly(alkylene glycol) chain (e.g., -OH).

In exemplary embodiments of the present invention, the reactive group comprises a carboxylate (e.g., an activated carboxylate such as an N-hydroxysuccinimide ester).

Optionally, the reactive group reacts with an amine group in the protein (e.g., in a lysine residue and/or an N-terminus) to form an amide bond.

In some embodiments, the reaction of the reactive group comprises reductive amination, wherein an amine group reacts with an aldehyde group to form an imine, and the imine is reduced (e.g., by addition of a reducing agent, such as sodium cyanoborohydride) to form an amine bond. The reactive group may be an amine group which reacts with an aldehyde group of the protein (e.g., on a saccharide moiety attached to the polypeptide of the protein), or the reactive group may be an aldehyde group which reacts with an amine group of the protein (e.g., on a lysine residue). Optionally, a saccharide moiety of a protein (i.e., a glycan) is oxidized by an oxidizing agent to form an aldehyde group, prior to reaction of the reactive group with the protein. For example, reaction of a saccharide with sodium periodate may be used to produce a pair of aldehyde groups in a saccharide moiety.

In some embodiments, at least one of the reactive groups is selected so as to react with one functionality of a protein monomer (e.g., an amine group of a lysine residue or N-terminus), and at least one of the reactive groups is selected so as to react with a different functionality of a protein monomer (e.g., a thiol group of a cysteine residue).

As used herein, the terms "amine" and "amino" refer to either a -NR'R" group, wherein R' and R" are selected from the group consisting of hydrogen, alkyl, cycloalkyl, heteroalicyclic (bonded through a ring carbon), aryl and heteroaryl (bonded through a ring carbon). R' and R" are bound via a carbon atom thereof. Optionally, R' and R" are selected from the group consisting of hydrogen and alkyl comprising 1 to 4 carbon atoms. Optionally, R' and R" are hydrogen.

As used herein throughout, the term "alkyl" refers to a saturated aliphatic hydrocarbon including straight chain and branched chain groups. Preferably, the alkyl group has 1 to 20 carbon atoms. Whenever a numerical range; *e.g.,* "1-20", is stated herein, it implies that the group, in this case the alkyl group, may contain 1 carbon atom, 2 carbon atoms, 3 carbon atoms, etc., up to and including 20 carbon atoms. More preferably, the alkyl is a medium size alkyl having 1 to 10 carbon atoms. Most preferably, unless otherwise indicated, the alkyl is a lower alkyl having 1 to 4 carbon atoms. The alkyl group may be substituted or unsubstituted. When substituted, the substituent group can be, for example, cycloalkyl, alkenyl, alkynyl, aryl, heteroaryl, heteroalicyclic, halo, hydroxy, alkoxy, aryloxy, thiohydroxy, thioalkoxy, thioaryloxy, sulfinyl, sulfonyl, cyano, nitro, azide, phosphonyl, phosphinyl, oxo, carbonyl, thiocarbonyl, urea, thiourea, O-carbamyl, N-carbamyl, O-thiocarbamyl, N-thiocarbamyl, C-amido, N-amido, C-carboxy, O-carboxy, sulfonamido, and amino, as these terms are defined herein.

A "cycloalkyl" group refers to an all-carbon monocyclic or fused ring (*i.e.,* rings which share an adjacent pair of carbon atoms) group wherein one of more of the rings does not have a completely conjugated pi-electron system. Examples, without limitation, of cycloalkyl groups are cyclopropane, cyclobutane, cyclopentane, cyclopentene, cyclohexane, cyclohexadiene, cycloheptane, cycloheptatriene, and adamantane. A cycloalkyl group may be substituted or unsubstituted. When substituted, the substituent group can be, for example, alkyl, alkenyl, alkynyl, aryl, heteroaryl, heteroalicyclic, halo, hydroxy, alkoxy, aryloxy, thiohydroxy, thioalkoxy, thioaryloxy, sulfinyl, sulfonyl, cyano, nitro, azide, phosphonyl, phosphinyl, oxo, carbonyl, thiocarbonyl, urea, thiourea, O-carbamyl, N-carbamyl, O-thiocarbamyl, N-thiocarbamyl, C-amido, N-amido, C-carboxy, O-carboxy, sulfonamido, and amino, as these terms are defined herein.

An "alkenyl" group refers to an alkyl group which consists of at least two carbon atoms and at least one carbon-carbon double bond.

An "alkynyl" group refers to an alkyl group which consists of at least two carbon atoms and at least one carbon-carbon triple bond.

An "aryl" group refers to an all-carbon monocyclic or fused-ring polycyclic (*i.e.,* rings which share adjacent pairs of carbon atoms) groups having a completely conjugated pi-electron system. Examples, without limitation, of aryl groups are phenyl, naphthalenyl and anthracenyl. The aryl group may be substituted or unsubstituted. When substituted, the substituent group can be, for example, alkyl, alkenyl, alkynyl, cycloalkyl, aryl, heteroaryl, heteroalicyclic, halo, hydroxy, alkoxy, aryloxy, thiohydroxy, thioalkoxy, thioaryloxy, sulfinyl, sulfonyl, cyano, nitro, azide, phosphonyl, phosphinyl, oxo, carbonyl, thiocarbonyl, urea, thiourea, O-carbamyl, N-carbamyl, O-thiocarbamyl, N-thiocarbamyl, C-amido, N-amido, C-carboxy, O-carboxy, sulfonamido, and amino, as these terms are defined herein.

A "heteroaryl" group refers to a monocyclic or fused ring (i.e., rings which share an adjacent pair of atoms) group having in the ring(s) one or more atoms, such as, for example, nitrogen, oxygen and sulfur and, in addition, having a completely conjugated pi-electron system. Examples, without limitation, of heteroaryl groups include pyrrole, furane, thiophene, imidazole, oxazole, thiazole, pyrazole, pyridine, pyrimidine, quinoline, isoquinoline and purine. The heteroaryl group may be substituted or unsubstituted. When substituted, the substituent group can be, for example, alkyl, alkenyl, alkynyl, cycloalkyl, aryl, heteroaryl, heteroalicyclic, halo, hydroxy, alkoxy, aryloxy, thiohydroxy, thioalkoxy, thioaryloxy, sulfinyl, sulfonyl, cyano, nitro, azide, phosphonyl, phosphinyl, oxo, carbonyl, thiocarbonyl, urea, thiourea, O-carbamyl, N-carbamyl, O-thiocarbamyl, N-thiocarbamyl, C-amido, N-amido, C-carboxy, O-carboxy, sulfonamido, and amino, as these terms are defined herein.

A "heteroalicyclic" group refers to a monocyclic or fused ring group having in the ring(s) one or more atoms such as nitrogen, oxygen and sulfur. The rings may also have one or more double bonds. However, the rings do not have a completely conjugated pi-electron system. The heteroalicyclic may be substituted or unsubstituted. When substituted, the substituted group can be, for example, lone pair electrons, alkyl, alkenyl, alkynyl, cycloalkyl, aryl, heteroaryl, heteroalicyclic, halo, hydroxy, alkoxy, aryloxy, thiohydroxy, thioalkoxy, thioaryloxy, sulfinyl, sulfonyl, cyano, nitro, azide, phosphonyl, phosphinyl, oxo, carbonyl, thiocarbonyl, urea, thiourea, O-carbamyl, N-carbamyl, O-thiocarbamyl, N-thiocarbamyl, C-amido, N-amido, C-carboxy, O-carboxy, sulfonamido, and amino, as these terms are defined herein. Representative examples are piperidine, piperazine, tetrahydrofuran, tetrahydropyran, morpholine and the like.

A "hydroxy" group refers to an -OH group.

An "azide" group refers to a -N=N⁺=N⁻ group.

An "alkoxy" group refers to both an -O-alkyl and an -O-cycloalkyl group, as defined herein.

An "aryloxy" group refers to both an -O-aryl and an -O-heteroaryl group, as defined herein.

An "ether" refers to both an alkoxy and an aryloxy group, wherein the group is linked to an alkyl, alkenyl, alkynyl, cycloalkyl, aryl, heteroaryl or heteroalicyclic group.

An ether bond describes a -O- bond.

A "thiohydroxy" or "thiol" group refers to a -SH group.

A "thioalkoxy" group refers to both an -S-alkyl group, and an -S-cycloalkyl group, as defined herein.

A "thioaryloxy" group refers to both an -S-aryl and an -S-heteroaryl group, as defined herein.

A "thioether" refers to both a thioalkoxy and a thioaryloxy group, wherein the group is linked to an alkyl, alkenyl, alkynyl, cycloalkyl, aryl, heteroaryl or heteroalicyclic group.

A thioether bond describes a -S- bond.

A "disulfide" group refers to both a -S-thioalkoxy and a -S-thioaryloxy group. A disulfide bond describes a -S-S- bond.

A "carbonyl" group refers to a -C(=O)-R' group, where R' is defined as hereinabove.

A "thiocarbonyl" group refers to a -C(=S)-R' group, where R' is as defined herein.

A "carboxyl" refers to both "C-carboxy" and O-carboxy".

A "C-carboxy" group refers to a -C(=O)-O-R' groups, where R' is as defined herein.

An "O-carboxy" group refers to an R'C(=O)-O- group, where R' is as defined herein.

An "oxo" group refers to a =O group.

A "carboxylate" or "carboxyl" encompasses both C-carboxy and O-carboxy groups, as defined herein.

A "carboxylic acid" group refers to a C-carboxy group in which R' is hydrogen.

A "thiocarboxy" or "thiocarboxylate" group refers to both -C(=S)-O-R' and -O-C(=S)R' groups.

An "ester" refers to a C-carboxy group wherein R' is not hydrogen.

An ester bond refers to a -O-C(=O)- bond.

A thioester bond refers to a -O-C(=S)- bond or to a -S-C(=O) bond.

A "halo" group refers to fluorine, chlorine, bromine or iodine.

A "sulfinyl" group refers to an -S(=O)-R' group, where R' is as defined herein.

A "sulfonyl" group refers to an -S(=O)₂-R' group, where R' is as defined herein.

A "sulfonate" group refers to an -S(=O)₂-O-R' group, where R' is as defined herein.

A "sulfate" group refers to an -O-S(=O)₂-O-R' group, where R' is as defined as herein.

A "sulfonamide" or "sulfonamido" group encompasses both S-sulfonamido and N-sulfonamido groups, as defined herein.

An "S-sulfonamido" group refers to a -S(=O)₂-NR'R" group, with each of R' and R" as defined herein.

An "N-sulfonamido" group refers to an R'S(=O)₂-NR" group, where each of R' and R" is as defined herein.

An "O-carbamyl" group refers to an -OC(=O)-NR'R" group, where each of R' and R" is as defined herein.

An "N-carbamyl" group refers to an R'OC(=O)-NR"- group, where each of R' and R" is as defined herein.

A "carbamyl" or "carbamate" group encompasses O-carbamyl and N-carbamyl groups.

A carbamate bond describes a -O-C(=O)-NR'- bond, where R' is as described herein.

An "O-thiocarbamyl" group refers to an -OC(=S)-NR'R" group, where each of R' and R" is as defined herein.

An "N-thiocarbamyl" group refers to an R'OC(=S)NR"- group, where each of R' and R" is as defined herein.

A "thiocarbamyl" or "thiocarbamate" group encompasses O-thiocarbamyl and N-thiocarbamyl groups.

A thiocarbamate bond describes a -O-C(=S)-NR'- bond, where R' is as described herein.

A "C-amido" group refers to a -C(=O)-NR'R" group, where each of R' and R" is as defined herein.

An "N-amido" group refers to an R'C(=O)-NR"- group, where each of R' and R" is as defined herein.

An "amide" group encompasses both C-amido and N-amido groups.

An amide bond describes a -NR'-C(=O)- bond, where R' is as defined herein.

An amine bond describes a bond between a nitrogen atom in an amine group (as defined herein) and an R' group in the amine group.

A thioamide bond describes a -NR'-C(=S)- bond, where R' is as defined herein.

A "urea" group refers to an -N(R')-C(=O)-NR"R"' group, where each of R' and R" is as defined herein, and R'" is defined as R' and R" are defined herein.

A "nitro" group refers to an -NO₂ group.

A "cyano" group refers to a -C=N group.

The term "phosphonyl" or "phosphonate" describes a -P(=O)(OR')(OR") group, with R' and R" as defined hereinabove.

The term "phosphate" describes an -O-P(=O)(OR')(OR") group, with each of R' and R" as defined hereinabove.

A "phosphoric acid" is a phosphate group is which each of R is hydrogen.

The term "phosphinyl" describes a -PR'R" group, with each of R' and R" as defined hereinabove.

The term "thiourea" describes a -N(R')-C(=S)-NR"- group, with each of R' and R" as defined hereinabove.

As described herein, multimeric protein structures described herein may exhibit improved stability and longer lasting activity of a therapeutic protein *in vivo.* Such multimeric protein structures are therefore highly beneficial for use in various medical applications in which an activity of a therapeutic protein is desirable, including therapeutic and research applications.

Hence, according to some embodiments, the multimeric protein structure described herein is for use as a medicament. Optionally, the medicament is for treating a disease or disorder treatable by the therapeutic protein upon which the multimeric protein structure is based.

For example, in some embodiments, the medicament is for treating a disease or disorder associated with cell signaling, which may be treated by an activity of a therapeutic protein which is associated with cell signaling.

Exemplary diseases and disorders include, but are not limited to, a proliferative disease or disorder, an inflammatory disease or disorder, a hyperuricemia, hypothyroidism, Kallman syndrome, polycystic ovarian syndrome, a hypothalamic suppression, a hypopituitarism, hypogonadism, an eating disorder, female athlete triad, amenorrhea, osteoporosis, a hyperprolactinemia, a gonadotropin deficiency, and infertility.

The disease or disorder which is treatable by the multimeric protein structure (e.g., by a medicament comprising the protein structure) will depend on the therapeutic protein on which the protein structure is based.

Thus, a disease or disorder treatable by TNF-α includes a disease or disorder in which immunostimulation is desirable. Optionally, the disease or disorder is characterized by an immunodeficiency (e.g., AIDS). Optionally, the disease or disorder is cancer.

In any of the methods and uses described herein, the multimeric structures described herein can be utilized either *per se,* or preferably, as a part of a pharmaceutical composition which further comprises a pharmaceutically acceptable carrier.

According to another aspect of embodiments of the invention, there is provided a pharmaceutical composition that comprises a multimeric protein structure as described herein and a pharmaceutically acceptable carrier.

As used herein a "pharmaceutical composition" refers to a preparation of one or more of the multimeric protein structures described herein, with other chemical components such as pharmaceutically acceptable and suitable carriers and excipients. The purpose of a pharmaceutical composition is to facilitate administration of a compound to an organism.

Hereinafter, the term "pharmaceutically acceptable carrier" refers to a carrier or a diluent that does not cause significant irritation to an organism and does not abrogate the biological activity and properties of the administered compound. Examples, without limitations, of carriers are: propylene glycol, saline, emulsions and mixtures of organic solvents with water, as well as solid (e.g., powdered) and gaseous carriers.

Herein the term "excipient" refers to an inert substance added to a pharmaceutical composition to further facilitate administration of a compound. Examples, without limitation, of excipients include calcium carbonate, calcium phosphate, various sugars and types of starch, cellulose derivatives, gelatin, vegetable oils and polyethylene glycols.

Techniques for formulation and administration of drugs may be found in "Remington's Pharmaceutical Sciences" Mack Publishing Co., Easton, PA, latest edition.

Pharmaceutical compositions of the present invention may be manufactured by processes well known in the art, e.g., by means of conventional mixing, dissolving, granulating, dragee-making, levigating, emulsifying, encapsulating, entrapping or lyophilizing processes.

Pharmaceutical compositions for use in accordance with the present invention thus may be formulated in conventional manner using one or more pharmaceutically acceptable carriers comprising excipients and auxiliaries, which facilitate processing of the multimeric protein structure into preparations which can be used pharmaceutically. Proper formulation is dependent upon the route of administration chosen.

For injection or infusion, the multimeric protein structures of embodiments of the invention may be formulated in aqueous solutions, preferably in physiologically compatible buffers such as Hank's solution, Ringer's solution, or physiological saline buffer.with or without organic solvents such as propylene glycol, polyethylene glycol.

For transmucosal administration, penetrants are used in the formulation. Such penetrants are generally known in the art.

For oral administration, the multimeric protein structures of the invention can be formulated readily by combining the multimeric protein structures with pharmaceutically acceptable carriers well known in the art. Such carriers enable the multimeric protein structures described herein to be formulated as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethylcellulose, sodium carbomethylcellulose; and/or physiologically acceptable polymers such as polyvinylpyrrolidone (PVP). If desired, disintegrating agents may be added, such as cross-linked polyvinyl pyrrolidone, agar, or alginic acid or a salt thereof such as sodium alginate.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions and suitable organic solvents or solvent mixtures. Dyestuffs or pigments may be added to the tablets or dragee coatings for identification or to characterize different combinations of doses of active multimeric protein structure.

Pharmaceutical compositions, which can be used orally, include push-fit capsules made of gelatin as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate and, optionally, stabilizers. In soft capsules, the multimeric protein structures may be dissolved or suspended in suitable liquids. In addition, stabilizers may be added. All formulations for oral administration should be in dosages suitable for the chosen route of administration.

For buccal administration, the compositions may take the form of tablets or lozenges formulated in conventional manner.

For administration by inhalation, the multimeric protein structures for use according to embodiments of the present invention are conveniently delivered in the form of an aerosol spray presentation (which typically includes powdered, liquified and/or gaseous carriers) from a pressurized pack or a nebulizer, with the use of a suitable propellant, e.g., dichlorodifluoromethane, trichlorofluoromethane, dichlorotetrafluoroethane or carbon dioxide. In the case of a pressurized aerosol, the dosage unit may be determined by providing a valve to deliver a metered amount. Capsules and cartridges of, e.g., gelatin for use in an inhaler or insufflator may be formulated containing a powder mix of the multimeric protein structures and a suitable powder base such as, but not limited to, lactose or starch.

The multimeric protein structures described herein may be formulated for parenteral administration, e.g., by bolus injection or continuous infusion. Formulations for injection or infusion may be presented in unit dosage form, e.g., in ampoules or in multidose containers with optionally, an added preservative. The compositions may be suspensions, solutions or emulsions in oily or aqueous vehicles, and may contain formulatory agents such as suspending, stabilizing and/or dispersing agents.

Pharmaceutical compositions for parenteral administration include aqueous solutions of the multimeric protein structure preparation in water-soluble form. Additionally, suspensions of the multimeric protein structures may be prepared as appropriate oily injection suspensions and emulsions (e.g., water-in-oil, oil-in-water or water-in-oil in oil emulsions). Suitable lipophilic solvents or vehicles include fatty oils such as sesame oil, or synthetic fatty acids esters such as ethyl oleate, triglycerides or liposomes. Aqueous injection suspensions may contain substances, which increase the viscosity of the suspension, such as sodium carboxymethyl cellulose, sorbitol or dextran. Optionally, the suspension may also contain suitable stabilizers or agents, which increase the solubility of the multimeric protein structures to allow for the preparation of highly concentrated solutions. Alternatively, the multimeric protein structures may be in powder form for constitution with a suitable vehicle, e.g., sterile, pyrogen-free water, before use.

The multimeric protein structure of embodiments of the present invention may also be formulated in rectal compositions such as suppositories or retention enemas, using, e.g., conventional suppository bases such as cocoa butter or other glycerides.

The pharmaceutical compositions herein described may also comprise suitable solid of gel phase carriers or excipients. Examples of such carriers or excipients include, but are not limited to, calcium carbonate, calcium phosphate, various sugars, starches, cellulose derivatives, gelatin and polymers such as polyethylene glycols.

Pharmaceutical compositions suitable for use in the context of the present invention include compositions wherein the active ingredients are contained in an amount effective to achieve the intended purpose. More specifically, a therapeutically effective amount means an amount of multimeric protein structures effective to prevent, alleviate or ameliorate symptoms of disease or prolong the survival of the subject being treated.

For any multimeric protein structures used in the methods of the invention, the therapeutically effective amount or dose can be estimated initially from activity assays in animals. For example, a dose can be formulated in animal models to achieve a circulating concentration range that includes the IC₅₀ as determined by activity assays (e.g., the concentration of the test protein structures, which achieves a half-maximal increase in a biological activity of the multimeric protein structure). Such information can be used to more accurately determine useful doses in humans.

In some embodiments, a therapeutically effective amount for the multimeric protein structures of embodiments of the present invention may range between about 1 µg/kg body weight and about 500 mg/kg body weight.

Toxicity and therapeutic efficacy of the multimeric protein structures described herein can be determined by standard pharmaceutical procedures in experimental animals, e.g., by determining the EC₅₀, the IC₅₀ and the LD₅₀ (lethal dose causing death in 50 % of the tested animals) for a subject protein structure. The data obtained from these activity assays and animal studies can be used in formulating a range of dosage for use in human.

The dosage may vary depending upon the dosage form employed and the route of administration utilized. The exact formulation, route of administration and dosage can be chosen by the individual physician in view of the patient's condition. (See e.g., Fingl et al., 1975, in "The Pharmacological Basis of Therapeutics", Ch. 1 p.1).

Dosage amount and interval may be adjusted individually to provide plasma levels of the active moiety which are sufficient to maintain the desired effects, termed the minimal effective concentration (MEC). The MEC will vary for each preparation, but can be estimated from *in vitro* data; e.g., the concentration necessary to achieve the desired level of activity *in vitro.* Dosages necessary to achieve the MEC will depend on individual characteristics and route of administration. HPLC assays or bioassays can be used to determine plasma concentrations.

Dosage intervals can also be determined using the MEC value. Preparations should be administered using a regimen, which maintains plasma levels above the MEC for 10-90 % of the time, preferably between 30-90 % and most preferably 50-90 %.

Depending on the severity and responsiveness of the condition to be treated, dosing can also be a single administration of a slow release composition described hereinabove, with course of treatment lasting from several days to several weeks or until cure is effected or diminution of the disease state is achieved.

The amount of a composition to be administered will, of course, be dependent on the subject being treated, the severity of the affliction, the manner of administration, the judgment of the prescribing physician, etc.

Compositions of the present invention may, if desired, be presented in a pack or dispenser device, such as an FDA (the U.S. Food and Drug Administration) approved kit, which may contain one or more unit dosage forms containing the active ingredient. The pack may, for example, comprise metal or plastic foil, such as, but not limited to a blister pack or a pressurized container (for inhalation). The pack or dispenser device may be accompanied by instructions for administration. The pack or dispenser may also be accompanied by a notice associated with the container in a form prescribed by a governmental agency regulating the manufacture, use or sale of pharmaceuticals, which notice is reflective of approval by the agency of the form of the compositions for human or veterinary administration. Such notice, for example, may be of labeling approved by the U.S. Food and Drug Administration for prescription drugs or of an approved product insert. Compositions comprising a multimeric protein structure of embodiments of the invention formulated in a compatible pharmaceutical carrier may also be prepared, placed in an appropriate container, and labeled for treatment of an indicated condition or diagnosis, as is detailed herein.

Thus, according to an embodiment of the present invention, depending on the selected multimeric protein structures, the pharmaceutical composition described herein is packaged in a packaging material and identified in print, in or on the packaging material, for use in the treatment of a condition in which the activity of the multimeric protein structure is beneficial, as described hereinabove.

As used herein the term "about" refers to ± 10 %

The terms "comprises", "comprising", "includes", "including", "having" and their conjugates mean "including but not limited to".

The term "consisting of means "including and limited to".

The word "exemplary" is used herein to mean "serving as an example, instance or illustration". Any embodiment described as "exemplary" is not necessarily to be construed as preferred or advantageous over other embodiments and/or to exclude the incorporation of features from other embodiments.

The word "optionally" is used herein to mean "is provided in some embodiments and not provided in other embodiments". Any particular embodiment of the invention may include a plurality of "optional" features unless such features conflict.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention.

### MATERIALS AND METHODS

### Materials:

Actinomycin D (cat. # A1410) was obtained from Sigma, and stored at -80 °C while being protected from light;
bis-*N*-hydroxysuccinimide-poly(ethylene glycol) (bis-NHS-PEG) was obtained from Quanta Biodesign in PEG₅, PEG₉ and PEG₂₁ forms;
Dimethyl sulfoxide was obtained from Sigma;
MTT (dimethyl thiazolyl diphenyl tetrazolium salt) was obtained from Sigma, and stored at room temperature while being protected from light;
TNF-α (#CYT-223), having SEQ ID NO: 1, was obtained from ProSpec.
***ELISA assay:***
TNF-α (cross-linked or non-cross-linked) was diluted with phosphate buffer to a concentration of 10 µg/ml. 100 µL of the TNF-α solution was placed in 4 low-binding Eppendorf tubes. The samples were kept at 37 °C or at 4 °C for the indicated time periods.

The wells of a MaxiSorp-surface 96-well plate (Nunc F96 MicroWell, cat. 442404) were coated with 20 ng/mL of Enbrel^{®} diluted in carbonate bicarbonate buffer (pH 9.6) by overnight incubation at 4 °C. Between each step, the plate was washed 4 times (300 µL/well) with ELISA wash buffer (KPL) diluted 1:40 in double-distilled water. Blocking was performed by incubation with Blocker Casein in PBS (Thermo Scientific) for 1 hour at room temperature.

TNF-α was diluted with 1 % bovine serum albumin dilution buffer (KPL) to a TNF-α concentration of 0.25-100 ng/mL and was used to construct a standard curve. The samples were diluted with the same dilution buffer by 1:1000 and by 1:10,000. The TNF-α standards and the tested samples were incubated in the plate for 2 hours at room temperature with shaking.

TNF-α was detected by biotinylated anti-TNF antibody (part of an Hbt Human TNF-α ELISA Kit, product # HK307, HyCult Biotechnologies), diluted 1:12. Biotin was detected by horseradish peroxidase (HRP)-conjugated streptavidin (part of the Hbt Human TNF-α ELISA Kit). Color was developed by adding TMB (3,3',5,5'-tetramethylbenzidine) as a chromogenic substrate, and stopping the reaction by HCl. Results were calculated according to the TNF-α standard curve.

### EXAMPLE 1

### Effect of cross-linking on activity and stability of TNF-α

TNF-α was reacted with bis-N-hydroxysuccinimide-poly(ethylene glycol) (bis-NHS-PEG) cross-linking reagents. In order to assess the effect of cross-linker length, bis-NHS-PEG reagents of various lengths (bis-NHS-PEG₅, bis-NHS-PEG₉, bis-NHS-PEG₂₁) were used.

For each reaction, 100 molar equivalents of the bis-NHS-PEG in 2 µl DMSO were added to 20 µg of human TNF-α in 20 µl of phosphate buffer (pH 8). The reaction mixture was kept at room temperature for 2 hours. The reaction products were then analyzed by SDS-PAGE analysis.

As shown in Figure 1, native TNF-α existed primarily in a monomeric form under the SDS-PAGE conditions, whereas TNF-α cross-linked with bis-NHS-PEG existed primarily in a trimeric form. As further shown therein, reaction with bis-NHS-PEG increased the molecular weight of the TNF-α.

These results indicate that the TNF-α was covalently cross-linked by each of the tested bis-NHS-PEG species.

The biological activity of the cross-linked TNF-α was assessed *in vitro,* by determining the effect of cross-linked TNF-α on the viability of mouse L929 fibroblasts.

L929 cells were plated in a 96-well assay plate (Costar) by placing 100 µL per well of assay medium (RPMI supplemented with 2 % fetal bovine serum) with 2.5 x 10⁵/mL cells. The cells were incubated overnight at 37 °C in a 5 % CO₂ atmosphere. 50 µL per well of cross-linked or non-cross-linked TNF-α in assay medium was then added, to achieve a final TNF-α concentration ranging from 0.3125 to 10 ng/mL, and the cells were incubated for a further 2 hours at 37 °C. Control wells contained cells without TNF-α. The medium was then removed from the wells. 50 µL of a solution of 4 µg/mL actinomycin D in assay medium was then added to each well, and the cells were then incubated for 24 hours at 37 °C.

Viability was measured using a MTT (dimethyl thiazolyl diphenyl tetrazolium salt) colorimetric assay. Medium was removed from the wells, and the cells were then incubated for 2 hours with 100 µL per well of a 0.45 mg/mL solution of MTT in assay medium. The MTT solution was then removed and 50 µL of a lysing solution (20 % sodium dodecyl sulfate and 50 % dimethyl formamide) was added to each well for 10-30 minutes, shaken and mixed. The absorption of each well at a wavelength of 570-650 nm was then measured.

As shown in Figure 2, TNF-α cross-linked with bis-NHS-PEG₅ was approximately as cytotoxic as non-cross-linked TNF-α, whereas TNF-α cross-linked with bis-NHS-PEG₉ was less cytotoxic, and TNF-α cross-linked with bis-NHS-PEG₂₁ did not exhibit cytotoxicity.

These results indicate that cross-linking TNF-α with short cross-linkers such as PEG₅ preserves the biological activity of TNF-α, whereas cross-linking with longer cross-linkers interferes with the biological activity of TNF-α.

The stability of TNF-α activity under physiological conditions was evaluated by storing samples of TNF-α cross-linked with bis-NHS-PEG₅ and samples of non-cross-linked TNF-α in phosphate buffer (100 mM) at pH 7.4, for 2 or 8 hours, or overnight (at 4 °C or at 37 °C). TNF-α activity was then determined by an ELISA assay using etanercept (Enbrel^{®}), a TNF-α receptor, as described in the Materials and Methods section hereinabove. Wells were coated with etanercept, contacted with the TNF-α sample, and then washed.

As shown in Figure 3, TNF-α cross-linked with bis-NHS-PEG₅ exhibited a small (-20 %) decrease in activity when stored overnight at 37 °C relative to the activity of the same protein when overnight at 4 °C, whereas non-cross-linked TNF-α exhibited a considerably larger (-80 %) decrease in activity. As further shown therein, cross-linked TNF-α stored for 2 or 8 hours at 37 °C exhibited the same activity as cross-linked TNF-α stored at 4 °C overnight, whereas non-cross-linked TNF-α exhibited a significant decrease in activity after even 2 hours at 37 °C.

These results indicate that cross-linking TNF-α results in a considerably longer lasting activity under physiological conditions (pH 7.4, 37 °C).

## Claims

1. A multimeric protein structure comprising at least two monomers of a therapeutic protein, said monomers being covalently linked to one another via a non-peptidic linking moiety, the multimeric protein structure featuring a characteristic selected from the group consisting of:
(a) a biological activity upon subjecting the multimeric protein structure to physiological conditions for one day, which is at least 10 % higher than an activity of a native form of said therapeutic protein upon subjecting said native form of said protein to said physiological conditions for one day;
(b) a biological activity which decreases upon subjecting the multimeric protein structure to physiological conditions for one day by a percentage which is at least 10 % less than the percentage by which an activity of said native form of said therapeutic protein decreases upon subjecting said native form of said protein to said physiological conditions for one day; and
(c) a circulating half-life in a physiological system which is higher by at least 20 % than a circulating half-life of said native form of said protein,
said therapeutic protein being selected from the group consisting of a TNF-α and fragments thereof, and said biological activity being binding to a TNF-α receptor.
wherein said at least one non-peptidic linking moiety has a general formula:
-X₁-(CR₁R₂-CR₃R₄-Y)n-X₂-
wherein each of X₁ and X₂ is a functional group that forms a covalent bond with at least one of said monomers;
Y is O, S or NR₅;
n is 3, 4, 5, 6, 7 or 8; and
each of R₁, R₂, R₃, R₄ and R₅ is independently selected from the group consisting of hydrogen, alkyl, cycloalkyl, alkenyl, alkynyl, alkoxy, hydroxy, oxo, thiol and thioalkoxy.

2. A multimeric protein structure comprising at least two monomers of a therapeutic protein, said monomers being covalently linked to one another via a bifunctional non-peptidic linking moiety comprising a poly(alkylene glycol), said linking moiety being no more than 30 atoms long, said therapeutic protein is selected from the group consisting of a TNF-α and fragments thereof.

3. The multimeric protein structure of claim 2, featuring a characteristic selected from the group consisting of:
(a) a biological activity upon subjecting the multimeric protein structure to physiological conditions for one day, which is at least 10 % higher than an activity of a native form of said therapeutic protein upon subjecting said native form of said protein to said physiological conditions for one day;
(b) a biological activity which decreases upon subjecting the multimeric protein structure to physiological conditions for one day by a percentage which is at least 10 % less than the percentage by which an activity of said native form of said therapeutic protein decreases upon subjecting said native form of said protein to said physiological conditions for one day; and
(c) a circulating half-life in a physiological system which is higher by at least 20 % than a circulating half-life of said native form of said protein,
said biological activity being binding to a TNF-α receptor.

4. The multimeric protein structure of any of claims 1 to 3, wherein a biological activity of said multimeric protein structure remains substantially unchanged upon subjecting the multimeric protein structure to physiological conditions for 8 hours, said biological activity being binding to a TNF-α receptor.

5. The multimeric protein structure of any of claims 1 to 4, wherein said therapeutic protein is a TNF-α.

6. The multimeric protein structure of claim 2, wherein said poly(alkylene glycol) comprises two functional groups, each functional group forming a covalent bond with one of said monomers.

7. The multimeric protein structure of claim 6, wherein said two functional groups are terminal groups of said poly(alkylene glycol).

8. The multimeric protein structure of any of claims 2, 6 and 7, wherein said at least one linking moiety has a general formula:
-X₁-(CR₁R₂-CR₃R₄-Y)n-X₂-
wherein each of X₁ and X₂ is a functional group that forms a covalent bond with at least one of said monomers;
Y is O, S or NR₅;
n is 3, 4, 5, 6, 7, 8 or 9; and
each of R₁, R₂, R₃, R₄ and R₅ is independently selected from the group consisting of hydrogen, alkyl, cycloalkyl, alkenyl, alkynyl, alkoxy, hydroxy, oxo, thiol and thioalkoxy.

9. The multimeric protein structure of any of claims 6 to 8, wherein at least one of said functional groups forms an amide bond with a monomer of said therapeutic protein.

10. A pharmaceutical composition comprising the multimeric protein structure of any of claims 1 to 9 and a pharmaceutically acceptable carrier.

11. The multimeric protein structure of any of claims 1 to 9, for use in treating a disease or disorder treatable by said therapeutic protein.

12. A process of preparing the multimeric protein structure of any of claims 1 to 9, the process comprising reacting said therapeutic protein with a cross-linking agent which comprises said linking moiety and at least two reactive groups.

13. The process of claim 12, wherein a molar ratio of said cross-linking agent to monomers of said therapeutic protein is in a range of from 5:1 to 500:1.

## Patentansprüche

1. Multimere Proteinstruktur enthaltend wenigstens zwei Monomere eines therapeutischen Proteins, wobei die Monomere miteinander kovalent über einen nicht-peptidischen Verbindungsrest miteinander verbunden sind, wobei die multimere Proteinstruktur ein Merkmal ausgestaltet, welches aus der Gruppe ausgewählt ist, welche besteht aus:
(a) einer biologischen Aktivität nach Unterwerfen der multimeren Proteinstruktur gegenüber physiologischen Bedingungen für einen Tag, welche wenigstens 10 % höher ist als die Aktivität einer nativen Form des therapeutischen Proteins nach Unterwerfen der nativen Form des Proteins gegenüber den physiologischen Bedingungen für einen Tag,
(b) einer biologischen Aktivität, welche nach Unterwerfen der multimeren Proteinstruktur gegenüber physiologischen Bedingungen für einen Tag um einen Prozentsatz abnimmt, welcher wenigstens 10 % niedriger ist als der Prozentsatz, um den die Aktivität der nativen Form des therapeutischen Proteins nach Unterwerfen der nativen Form des Proteins gegen über den physiologischen Bedingungen für einen Tag abnimmt, und
(c) einer zirkulierenden Halbwertszeit in einem physiologischen System, welche um 20 % höher ist als zirkulierende Halbwertszeit der nativen Form des Proteins,
wobei das therapeutische Protein aus der Gruppe ausgewählt ist, welche aus einem TNF-α und Fragmenten hiervon besteht, und, wobei die biologische Aktivität das Binden an einen TNF-α-Rezeptor ist,
wobei der wenigstens eine nicht-peptidische Verbindungsrest die folgende allgemeine Formel aufweist:
-X₁-(CR₁R₂-CR₃R₄-Y)ₙ-X₂-
wobei jeder von X₁ und X₂ eine funktionelle Gruppe ist, welche mit wenigstens einem der Monomere eine kovalente Bindung ausbildet,
Y O, S oder NR₅ ist,
n 3, 4, 5, 6, 7 oder 8 ist und
jeder von R₁, R₂, R₃, R₄ und R₅ unabhängig voneinander aus der Gruppe ausgewählt ist, welche aus Wasserstoff, Alkyl, Cycloalkyl, Alkenyl, Alkinyl, Alkoxy, Hydroxy, Oxo, Thiol und Thioalkoxy besteht.

2. Multimere Proteinstruktur enthaltend wenigstens zwei Monomere eines therapeutischen Proteins, wobei die Monomere miteinander über einen bifunktionellen nicht-peptidischen Verbindungsrest miteinander verbunden sind, welcher ein Polyalkylenglycol enthält, wobei der Verbindungsrest nicht mehr als 30 Atome lang ist, wobei das therapeutische Protein aus der Gruppe ausgewählt ist, welche aus einem TNF-α und Fragmenten hiervon besteht.

3. Multimere Proteinstruktur nach Anspruch 2, welche ein Merkmal ausgestaltet, das aus der Gruppe ausgewählt ist, welche besteht aus:
(a) einer biologischen Aktivität nach Unterwerfen der multimeren Proteinstruktur gegenüber physiologischen Bedingungen für einen Tag, welche wenigstens 10 % höher ist als die Aktivität einer nativen Form des therapeutischen Proteins nach Unterwerfen der nativen Form des Proteins gegenüber den physiologischen Bedingungen für einen Tag,
(b) einer biologischen Aktivität, welche nach Unterwerfen der multimeren Proteinstruktur gegenüber physiologischen Bedingungen für einen Tag um einen Prozentsatz abnimmt, welcher wenigstens 10 % niedriger ist als der Prozentsatz, um den die Aktivität der nativen Form des therapeutischen Proteins nach Unterwerfen der nativen Form des Proteins gegen über den physiologischen Bedingungen für einen Tag abnimmt, und
(c) einer zirkulierenden Halbwertszeit in einem physiologischen System, welche um 20 % höher ist als zirkulierende Halbwertszeit der nativen Form des Proteins,
wobei die biologische Aktivität das Binden an einen TNF-α-Rezeptor ist.

4. Multimere Proteinstruktur nach einem der Ansprüche 1 bis 3, wobei die biologische Aktivität der muiltimeren Proteinstruktur beim Unterwerfen der multimeren Proteinstruktur gegenüber physiologischen Bedingungen für 8 Stunden, wobei die biologische Aktivität das Binden an einen TNF-α-Rezeptor ist, im Wesentlichen unverändert verbleibt.

5. Multimere Proteinstruktur nach einem der Ansprüche 1 bis 4, wobei das therapeutische Protein TNF-α ist.

6. Multimere Proteinstruktur nach Anspruch 2, wobei das Polyalkylenglycol zwei funktionelle Gruppen umfasst, wobei jede funktionelle Gruppe mit einem der Monomere eine kovalente Bindung ausbildet.

7. Multimere Proteinstruktur nach Anspruch 6, wobei die beiden funktionellen Gruppen terminale Gruppen des Polyalkylenglycols sind.

8. Multimere Proteinstruktur nach einem der Ansprüche 2, 6 und 7, wobei wenigstens ein Verbindungsrest die folgende allgemeine Formel aufweist:
-X₁-(CR₁R₂-CR₃R₄-Y)ₙ-X₂-
wobei jeder von X₁ und X₂ eine funktionelle Gruppe ist, welche mit wenigstens einem der Monomere eine kovalente Bindung ausbildet,
Y O, S oder NR₅ ist,
n 3, 4, 5, 6, 7, 8 oder 9 ist und
jeder von R₁, R₂, R₃, R₄ und R₅ unabhängig voneinander aus der Gruppe ausgewählt ist, welche aus Wasserstoff, Alkyl, Cycloalkyl, Alkenyl, Alkinyl, Alkoxy, Hydroxy, Oxo, Thiol und Thioalkoxy besteht.

9. Multimere Proteinstruktur nach einem der Ansprüche 6 bis 8, wobei wenigstens eine der funktionellen Gruppen mit einem Monomer des therapeutischen Proteins eine Amidbindung ausbildet.

10. Pharmazeutische Zusammensetzung enthaltend eine multimere Proteinstruktur nach einem der Ansprüche 1 bis 9 und einen pharmazeutisch akzeptablen Träger.

11. Multimere Proteinstruktur nach einem der Ansprüche 1 bis 9 zur Verwendung bei der Behandlung einer Erkrankung oder Störung, welche durch das therapeutische Protein behandelbar ist.

12. Verfahren zum Herstellen einer multimeren Proteinstruktur nach einem der Ansprüche 1 bis 9, wobei das Verfahren das Reagieren des therapeutischen Proteins mit einem Vernetzungsmittel umfasst, welches den Verbindungsrest und wenigstens zwei reaktive Gruppen umfasst.

13. Verfahren nach Anspruch 12, wobei das molare Verhältnis des Vernetzungsmittels zu Monomeren des therapeutischen Proteins in einem Bereich von 5:1 bis 500:1 liegt.

## Revendications

1. Structure protéique multimère comprenant au moins deux monomères d'une protéine thérapeutique, lesdits monomères étant liés par liaison covalente l'un à l'autre par l'intermédiaire d'une fraction de liaison non peptidique, la structure protéique multimère présentant une caractéristique sélectionnée dans le groupe constitué :
(a) d'une activité biologique, lors de la soumission de la structure protéique multimère à des conditions physiologiques pendant un jour, qui est supérieure d'au moins 10 % à une activité d'une forme native de ladite protéine thérapeutique lors de la soumission de ladite forme native de ladite protéine auxdites conditions physiologiques pendant un jour ;
(b) d'une activité biologique qui diminue, lors de la soumission de la structure protéique multimère à des conditions physiologiques pendant un jour, d'un pourcentage qui est inférieur d'au moins 10 % au pourcentage de diminution d'une activité de ladite forme native de ladite protéine thérapeutique lors de la soumission de ladite forme native de ladite protéine auxdites conditions physiologiques pendant un jour ; et
(c) d'une demi-vie circulante dans un système physiologique qui est supérieure d'au moins 20 % à une demi-vie circulante de ladite forme native de ladite protéine,
ladite protéine thérapeutique étant sélectionnée dans le groupe constitué d'un TNF-α et de fragments de celui-ci, et ladite activité biologique étant la liaison à un récepteur du TNT-α ;
dans laquelle ladite ou lesdites fractions de liaison non peptidique ont une formule générale :
**-X₁-(CR₁R₂-CR₃R₄-Y)ₙ-X₂-**
dans laquelle X₁ et X₂ sont chacun un groupe fonctionnel qui forme une liaison covalente avec au moins l'un desdits monomères ;
Y est 0, S ou NR₅ ;
n vaut 3, 4, 5, 6, 7 ou 8 ; et
chacun de R₁, R₂, R₃, R₄ et R₅ est indépendamment sélectionné dans le groupe constitué d'un atome d'hydrogène et des groupes alkyle, cycloalkyle, alcényle, alcynyle, alcoxy, hydroxy, oxo, thiol et thioalcoxy.

2. Structure protéique multimère comprenant au moins deux monomères d'une protéine thérapeutique, lesdits monomères étant liés par liaison covalente l'un à l'autre par l'intermédiaire d'une fraction de liaison non peptidique bifonctionnelle comprenant un poly(alkylène glycol), ladite fraction de liaison n'étant pas d'une longueur supérieure à 30 atomes, ladite protéine thérapeutique est sélectionnée dans le groupe constitué du TNF-α et de fragments de celui-ci.

3. Structure protéique multimère selon la revendication 2, présentant une caractéristique sélectionnée dans le groupe constitué :
(a) d'une activité biologique, lors de la soumission de la structure protéique multimère à des conditions physiologiques pendant un jour, qui est supérieure d'au moins 10 % à une activité d'une forme native de ladite protéine thérapeutique lors de la soumission de ladite forme native de ladite protéine auxdites conditions physiologiques pendant un jour ;
(b) d'une activité biologique qui diminue, lors de la soumission de la structure protéique multimère à des conditions physiologiques pendant un jour, d'un pourcentage qui est inférieur d'au moins 10 % au pourcentage de diminution d'une activité de ladite forme native de ladite protéine thérapeutique lors de la soumission de ladite forme native de la protéine auxdites conditions physiologiques pendant un jour ; et
(c) d'une demi-vie circulante dans un système physiologique qui est supérieure d'au moins 20 % à une demi-vie circulante de ladite forme native de ladite protéine,
ladite activité biologique étant la liaison à un récepteur du TNF-α.

4. Structure protéique multimère selon l'une quelconque des revendications 1 et 3, dans laquelle une activité biologique de ladite structure protéique multimère reste sensiblement identique lors de la soumission de la structure protéique multimère à des conditions physiologiques pendant 8 heures, ladite activité biologique étant la liaison à un récepteur du TNF-α.

5. Structure protéique multimère selon l'une quelconque des revendications 1 à 4, dans laquelle ladite protéine thérapeutique est un TNF-α.

6. Structure protéique multimère selon la revendication 2, dans laquelle ledit poly(alkylène glycol) comprend deux groupes fonctionnels, chaque groupe fonctionnel formant une liaison covalente avec l'un desdits monomères.

7. Structure protéique multimère selon la revendication 6, dans laquelle lesdits deux groupes fonctionnels sont des groupes terminaux dudit poly(alkylène glycol).

8. Structure protéique multimère selon l'une quelconque des revendications 2, 6 et 7, dans laquelle au moins une fraction de liaison a une formule générale :
**-X₁-(CR₁R₂-CR₃R₄-Y)n-X₂-**
dans laquelle X₁ et X₂ sont chacun un groupe fonctionnel qui forme une liaison covalente avec au moins l'un desdits monomères ;
Y est 0, S ou NR₅ ;
n vaut 3, 4, 5, 6, 7, 8 ou 9 ; et
chacun de R₁, R₂, R₃, R₄ et R₅ est indépendamment sélectionné dans le groupe constitué d'un atome d'hydrogène et des groupes alkyle, cycloalkyle, alcényle, alcynyle, alcoxy, hydroxy, oxo, thiol et thioalcoxy.

9. Structure protéique multimère selon l'une quelconque des revendications 6 à 8, dans laquelle au moins l'un desdits groupes fonctionnels forme une liaison amide avec un monomère de ladite protéine thérapeutique.

10. Composition pharmaceutique comprenant la structure protéique multimère selon l'une quelconque des revendications 1 à 9, et un vecteur pharmaceutiquement acceptable.

11. Structure protéique multimère selon l'une quelconque des revendications 1 à 9, pour son utilisation dans le traitement d'une maladie ou d'un trouble pouvant être traité par ladite protéine thérapeutique.

12. Procédé de préparation de la structure protéique multimère selon l'une quelconque des revendications 1 à 9, le procédé comprenant la mise en réaction de ladite protéine thérapeutique avec un agent de réticulation qui comprend ladite fraction de liaison et au moins deux groupes réactifs.

13. Procédé selon la revendication 12, dans lequel un rapport molaire dudit agent de réticulation sur les monomères de ladite protéine thérapeutique est situé dans une plage allant de 5/1 à 500/1.


### SEQUENCE LISTING

<110> Protalix Ltd.
   Ruderfer, Ilya
   Shilovittzky, Orit
   Shulman, Avidor
   Shaaltiel, Yoseph
   Ben-Moshe, Tehila
   Shekhter, Talia
   Azulay, Yaniv
<120> MULTIMERIC FORMS OF THERAPEUTIC PROTEINS AND USES THEREOF
<130> 50865
<150> US 61/309,487
   <151> 2010-03-02
<160> 1
<170> PatentIn version 3.5
<210> 1
   <211> 158
   <212> PRT
   <213> Artificial sequence
<220>
   <223> biTNF-alpha recombinant polypeptide
<400> 1
   Met Val Arg Ser Ser Ser Arg Thr Pro Ser Asp Lys Pro Val Ala His
   1               5                   10                  15
   Val Val Ala Asn Pro Gln Ala Glu Gly Gln Leu Gln Trp Leu Asn Arg
               20                  25                  30
   Arg Ala Asn Ala Leu Leu Ala Asn Gly Val Glu Leu Arg Asp Asn Gln
           35                  40                  45
   Leu Val Val Pro Ser Glu Gly Leu Tyr Leu Ile Tyr Ser Gln Val Leu
       50                  55                  60
   Phe Lys Gly Gln Gly Cys Pro Ser Thr His Val Leu Leu Thr His Thr
   65                  70                  75                  80
   Ile Ser Arg Ile Ala Val Ser Tyr Gln Thr Lys Val Asn Leu Leu Ser
                   85                  90                  95
   Ala Ile Lys Ser Pro Cys Gln Arg Glu Thr Pro Glu Gly Ala Glu Ala
               100                 105                 110
   Lys Pro Trp Tyr Glu Pro Ile Tyr Leu Gly Gly Val Phe Gln Leu Glu
           115                 120                 125
   Lys Gly Asp Arg Leu Ser Ala Glu Ile Asn Arg Pro Asp Tyr Leu Asp
       130                 135                 140
   Phe Ala Glu Ser Gly Gln Val Tyr Phe Gly Ile Ile Ala Leu
   145                 150                 155